(19) Europäisches Patentamt
European Patent Office
Office européen des brevets

(11) Publication number: 0 029 130 B1

(12) EUROPEAN PATENT SPECIFICATION

(45) Date of publication of patent specification: 04.12.85

(51) Int. Cl.[4]: C 07 D 487/04, A 61 K 31/50 // (C07D487/04, 237:00, 249:00)

(21) Application number: 80106450.2

(22) Date of filing: 23.10.80

# (54) 3,6,7,8-Substituted-s-triazolo(4,3-b)-pyridazines, their preparation and compositions comprising them.

(30) Priority: 29.10.79 US 89071

(43) Date of publication of application: 27.05.81 Bulletin 81/21

(45) Publication of the grant of the patent: 04.12.85 Bulletin 85/49

(84) Designated Contracting States: AT BE CH DE FR IT LI LU NL SE

(56) References cited:
DE-A-2 741 763
US-A-3 708 484
US-A-3 915 968
US-A-3 948 912
US-A-4 136 182

(73) Proprietor: THE DOW CHEMICAL COMPANY
2030 Dow Center Abbott Road P.O. Box 1967
Midland, MI 48640 (US)

(72) Inventor: Peet, Norton Paul
5170 North Walden Lane
Indianapolis Indiana (US)
Inventor: Sunder, Shyam
1701 Harfield Drive 247
Indianapolis Indiana (US)

(74) Representative: Sgarbi, Renato et al
GRUPPO LEPETIT S.p.A. Patent and Trademark
Department Via Roberto Lepetit, 8
I-20124 Milano (IT)

EP 0 029 130 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new pharmacologically active heterocyclic compounds. More particularly, the invention relates to 3,6,7,8-substituted *s*-triazolo[4,3-*b*]pyridazines corresponding to the formula

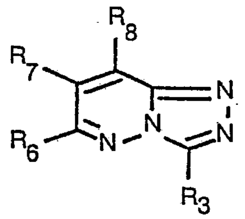

I

wherein $R_3$ represents hydrogen or loweralkyl. $R_6$ represents amino, loweralkylamino, diloweralkylamino, or heterocyclic amino or lower alkyl substituted heterocyclic amino, wherein the heterocyclic moiety forms a 5-, 6- or 7-membered ring, having one or two ring nitrogen atoms and zero or one ring sulfur or oxygen atom; and $R_7$ and $R_8$ taken together represent polymethylene or substituted polymethylene of 4 methylene units, e.g., $-CH_2-(CH_2)_3-$ substituted by loweralkyl, or methano or ethano bridges; and to pharmacologically acceptable salts of said compounds.

The compounds can be named either as *s*-triazolo[4,3-*b*]pyridazines or as 7,8,9,10-tetrahydro(1,2,4)-triazolo[3,4-*a*]phthalazines. When so named, the 7,8,9 and 10 positions refer to the carbons of the tetramethylene group attached to the pyridazine residue. The bridged compounds correspond to the formula

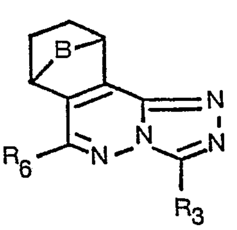

II

wherein $R_3$ and $R_6$ have the above significance, and B represents methylene or ethylene. Compounds of formula II can be named as substituted triazolopyridazines or as tetrahydrotriazolophthalazines.

In the present specification the terms "loweralkyl" and "lower alkoxy" refer to compounds containing one, two, three or four carbon atoms; and "halo" refers to fluoro, chloro or bromo.

The invention is inclusive of subgroups of compounds of the above formula, for example, those wherein $R_3$ is hydrogen; those wherein $R_3$ represents hydrogen, methyl, ethyl or propyl; those wherein $R_6$ is pyrrolidino; those wherein $R_6$ is piperidino; those wherein $R_6$ is morpholino or thiamorpholino; those wherein $R_6$ is amino; those wherein $R_6$ is azepinyl or diazepinyl; or those wherein $R_6$ is N-methyl piperazino; those wherein $R_7$ and $R_8$ are polymethylene; those wherein $R_7$ and $R_8$ are substituted polymethylene substituted by loweralkyl, methano or ethano, etc. Such subgroups are apparent from the above description and the following specification; and further listing is omitted for the sake of brevity.

Preferred groups of compounds comprises those wherein the loweralkyl is methyl; those wherein $R_3$ is hydrogen; those wherein $R_6$ is pyrrolidino, piperidino; 2-methylpyrrolidino, hexahydro-1H-azepin-1-yl, or 4-methyl-hexahydro-1H-1,4-diazepin-1-yl; and those wherein $R_3$ is hydrogen or methyl.

The compounds of the invention may be prepared by the reaction of a 3-halo-6-hydrazinopyridazine of formula III with a substituted carboxyl compound of formula IV

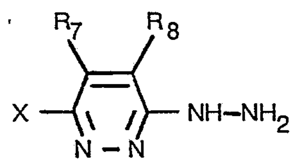

III

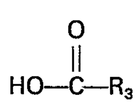

IV

wherein $R_3$, $R_7$ and $R_8$ have the above significance and wherein X is halo, generally at a temperature of 40° to 150°C, preferably 100° to 130°C, in an excess of acid optionally diluted with an inert solvent, having a boiling point of 40° to 200°C, preferably 75° to 175°C, followed by reacting the resulting 6-halo-triazolopyridazine with the corresponding $R_6$ amine, at a temperature of 75° to 160°C, preferably 100° to 140°C, in the same solvent employed for the reaction of compounds III and IV. Useful solvents include either an inert organic solvent or a hydroxylic solvent such as, for example, water, $C_1$—$C_4$ alkanols and their admixtures with water, $C_1$—$C_4$ alkoxy alkanols, glycol ethers, dioxane, aliphatic and cycloaliphatic

hydrocarbons, aromatic hydrocarbons, chlorobenzene and chlorinated aliphatic hydrocarbons such as methylene chloride. The reactions are preferably carried out at atmospheric pressure, although higher pressures may be employed if desired.

Thus, the compounds of the invention can be prepared by first reacting a 3-chloro-6-hydrazinopyridazine with an appropriate acid of formula IV. This reaction proceeds when the reactants are contacted and mixed, preferably at the boiling temperature of the reaction mixture. The first step of the reaction is preferably carried out in an excess of the reacting acid, the excess acid serving as reaction medium or component thereof. The 6-halo-triazolopyridazine product can be recovered from the reaction mixture by evaporation to remove the excess acid reaction medium and can be purified by conventional procedures such as recrystallization and washing .

The substituted 6-halo-triazolopyridazine, in an appropriate solvent, is then reacted with excess $R_6$ amine (e.g. at least two fold on a molar basis) or the $R_6$ amine in the presence of an inorganic base such as, for example, sodium carbonate or a non-nucleophilic amine, as hydrogen halide acceptor. The reaction is preferably carried out at the boiling temperature, using an excess of the $R_6$ amine as the reaction medium. The product is recovered by conventional procedures such as concentration under reduced pressure.

The starting materials for the above method can be prepared by procedures which are known. The necessary pyridazines for the method above are obtained by reacting the appropriate 3,6-dihalo-4,5-substituted pyridazine with hydrazine hydrate.

In an alternative procedure for preparing substituted triazolopyridazines of Formulae I and II, a 3,6-dihalo-4,5-substituted pyridazine of Formula V

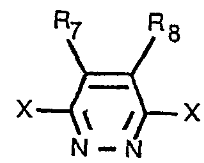

V

wherein X represents chloro, bromo or fluoro, is reacted with the $R_6$ amine to prepare a 3-halo pyridazine of Formula VI

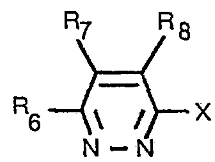

VI

The reaction is conveniently carried out in an inert solvent, as hereinbefore described, at a temperature of 25° to 150°C, preferably 50° to 140°C, in the presence of an inorganic base, such as, for example, sodium carbonate, a non-nucleophilic amine, or an excess of the $R_6$ amine, as a hydrogen halide acceptor.

The resulting 3-halopyridazine is then reacted with a lower alkanoyl hydrazine of Formula VII

$$R_3\overset{\overset{O}{\|}}{C}—NH—NH_2 \qquad VII$$

preferably in the presence of an acid catalyst, in an inert liquid medium, preferably an oxygenated solvent such as, for example, an alkylene glycol alkyl ether, at a temperature of from 50° to 200°C, preferably 100° to 175°C. The generation of hydrogen halide during the reaction accelerates the reaction rate. The product is recovered and purified by conventional procedures. These reactions are preferably carried out at atmospheric pressure although higher pressures may be employed if desired. In the above formulae, $R_3$, $R_6$, $R_7$ and $R_8$ have the significance set out above with respect to Formulae I—IV.

The hydrazino pyridazine starting materials can be prepared in analogy with known procedures. For example, 3,6-dichloro-4-methylpyridazine heated at reflux with excess hydrazine hydrate (50 percent in water) for 0.3 to 1 hour produces 3-chloro-4-methyl-6-hydrazinopyridazine and 3-chloro-5-methyl-6-hydrazinopyridazine. The isomers can be separated by fractional crystallization using ethanol as a solvent. See, Takahayashi, Pharm. Bull., *5,* 229 (1957); Chem. Abstr. *52:*6359, Linholter et al., Acta Chem. Scand. *16,* 2389 (1962); Chem. Abstr. *59:*1632g, Steck et al., J. Amer. Chem. Soc., *76,* 4454 (1954) and Horning et al., J. Org. Chem., *20,* 707 (1955).

When $R_7$ and $R_8$ are 1,3-cyclopentylene or 1,4-cyclohexylene, the dichloro intermediate is conveniently prepared from a 4,5,6,7-tetrahydro-4,7-(methano or ethano)-isobenzofuran-1,3-dione. Diels and Alder, Ann., 478, 149 (1930); Ann., 490, 236 (1931). The dione compound is reacted with excess hydrazine hydrate in an exothermic reaction to produce the corresponding hexahydro-5,8-(methano or ethano)phthalazine-1,4-dione. Additional heating at 100°—170°C for 10—30 minutes may be useful to complete the reaction. The phthalazine-1,4-dione is then reacted with excess phosphorus oxychloride heated at reflux for about 2—4 hours, cooled and the product hydrolyzed by careful addition of ice and water to produce the dichloro intermediate.

The 3,6,7,8-substituted-s-triazolo[4,3-*b*]pyridazine compounds corresponding to the above formulae and their pharmacologically acceptable salts have useful biological activity as bronchodilators and also have a desirably low toxicity and freedom from undersirable side effects at dosages consistent with good broncodilator activity. Some 6-acyclic or cyclic amino-*s*-triazolo[4,3-*b*]pyridazines are known from US patents 4,136,182 and 3,915,968 and from German Patent Application No. 2741763. They are stated to possess bronchodilating activity.

Other triazolopyridazines are known to have different properties. For example, 6-methyl-3-(4-morpholinyl)-8-phenyl-*s*-triazolo[4,3-*b*]pyridazine, rather than blocking histamine-induced bronchoconstriction, has been found to potentiate bronchoconstriction. The compound 6-morpholino-3-phenyl-*s*-triazolo[4,3-*b*]pyridazine, although a potent bronchodilator with a high $LD_{50}$ (low toxicity), has been found to produce audiogenic convulsions in laboratory animals at relatively low dosages. See U.S. Patent 4,136,182.

The triazolopyridazine compounds are crystalline solids which can be readily formulated in aqueous or alcoholic liquids. In general, the free base compounds are readily soluble in aqueous liquids, and the triazolopyridazine compounds are conveniently employed in either free base or salt form.

In practicing the method an effective bronchodilating amount of one or more substituted triazolopyridazine is administered internally to a mammal in need thereof by a route effective to bring the compound into contact with the bronchial and tracheal tissues of the mammal. Administration can be carried out either by a parenteral route, such as by intravenous, intraperitoneal, or intramuscular injection, or by introduction into the gastrointestinal tract via oral or rectal administration, for example, in order to bring about such contact via the blood stream, or by intratracheal administration, by inhalation of a solution in the form of a spray, for example.

The effective brochodilating amount of the compound, that is, the amount of the substituted triazolopyridazine sufficient to inhibit or alleviate bronchial spasm depends on various factors such as the size, type and age of the animal to be treated, the particular triazolopyridazine or pharmacologically-acceptable salt employed, the route and frequency of administration, the severity of spasm (if any) and the causative agent involved, and the time of administration. In particular cases, the dosage to be administered can be ascertained by conventional range finding techniques, for example, by observing the bronchodilator activity produced at different dosage rates. Good results can be obtained when the compound is administered at dosage rates from 1 to 3, to 10 to 50 milligrams of substituted triazolopyridazine compound per kilogram of animal body weight. It is generally desirable to administer individual dosages at the lowest amount which provides the desired protection from bronchial spasm consonant with a convenient dosing schedule. Dosage units adaptable to oral administration such as tablets, capsules, lozenges, elixirs, syrups and the like are preferred and the active triazolopyridazine compound can be formulated in conventional timed release capsule or tablet formulations.

Some of the compounds can produce audiogenic convulsant side effects at dosages which, though much higher than the effective dose for bronchodilation, are still below a toxic dosage. Brochodilator activity can be obtained at high, but non-toxic dosages at which additional factors could promote undesirable convulsant side effects, by eliminating other factors contributing to audiogenic convulsions. However, it is preferable to employ the compounds at effective dosages substantially below the audiogenic convulsant dosage, e.g. at one third, to one fifth, one-tenth or less of the audiogenic convulsant dosage. The audiogenic convulsant dosage, (dosage producing audiogenic convulsions) can be determined in known procedures, as described for example in U.S. Patent 4,136,182.

In practicing the method of the invention, the active ingredient is preferably incorporated in a composition comprising a pharmaceutical carrier and from about 5 to about 90 percent by weight of the substituted triazolopyridazine compond or a pharmacologically-acceptable salt thereof. The term "pharmaceutical carrier" refers to known pharmaceutical excipients useful in formulating pharmacologically-active compounds for internal administration to animals, and which are substantially non-toxic and non-sensitizing under conditions of use. The compositions can be prepared by known techniques for the preparation of tablets, capsules, lozenges, troches, suppositories, elixirs, syrups, emulsions, dispersions, wettable and effervescent powders, sterile injectable compositions, and can contain suitable excipients known to be useful in the preparation of the particular type of composition desired. Suitable pharmaceutical carriers and formulation techniques are found in standard texts, such as Remington's pharmaceutical Sciences, Mack Publishing Co., Easton, Pennsylvania.

As employed herein, the phrase "pharmacologically acceptable salt" refers to salts of the substituted triazolopyridazines, the anions of which are relatively nontoxic and innocuous to mammals at dosages consistent with good biological activity so that side effects ascribable to the anions do not vitiate the beneficial effects of the triazolopyridazine compounds. Suitable pharmacologically acceptable salts can be prepared by conventional procedures such as dissolving the free base compound in an inert organic solvent such as ether and treating the resulting solution with an excess ether solution of a suitable pharmacologically acceptable acid such as hydrochloric acid, or hydrobromic acid.

For the sake of brevity, such compounds will be hereinafter referred to simply as "triazolopyridazines".

The following examples illustrate the invention.

Example 1

1-Chloro-4-hydrazino-5,6,7,8-tetrahydro-5,8-methanophthalazine (25 grams; 0.12 mol) was mixed with 150 milliliters of formic acid and heated at the boiling temperature under reflux for 2 hours. The reaction mixture was concentrated by evaporation under reduced pressure and taken up in aqueous sodium bicarbonate. The resulting precipitate was collected by filtration, washed with water and dried in air. The 6-chloro-7,8,9,10-tetrahydro-7,10-methano[1,2,4]triazolo-[3,4-*a*]-phthalazine product (21.6 grams, 82.5% yield) was found to melt at 155°—156°C, and at 157°—158°C after recrystallization from a benzene-hexane mixture. The structure was confirmed by infrared spectroscopy, nuclear magnetic resonance analysis and elemental analysis. (Calculated for $C_{10}H_9ClN_4$: C, 54.42; H, 4.11; N, 25.39. Found: C, 54.40; H, 4.12; N, 25.20). This 6-chloro compound (6.5 grams, 0.029 mol) was mixed with 25 milliliters of N-methylpiperazine, and the mixture heated at boiling under reflux for 6 hours. The mixture was concentrated by evaporation under reduced pressure, then partitioned between methylene chloride and water. The organic layer was separated dried over sodium sulfate and concentrated to obtain the 7,8,9,10-tetrahydro-6-[4-methyl-1-piperazinyl]-7,10-methanol(1,2,4)triazolo[3,4-*a*]phthalazine product. After recrystallization from benzene/hexane, 6.6 grams (79 percent yield) of the product were obtained, with a melting point of 172°—173°C. Structure was confirmed by infrared and nuclear magnetic resonance analysis, and by elemental analysis. (Calculated for $C_{15}H_{20}N_6$: C, 63.35; H, 7.09; N, 29.56. Found: C, 63.30; H, 7.05; N, 29.33.)

Example 2

In a procedure similar to that of Example 1, 1-chloro-4-hydrazino-5,6,7,8-tetrahydro-5,8-methanophthalazine (25.0 grams, 0.119 mol) was mixed with 150 milliliters of acetic acid, and the mixture was heated at the boiling temperature under reflux for two hours. The reaction mixture was concentrated and the residue taken up in aqueous sodium bicarbonate. The resulting precipitate was collected, washed with water and air-dried to yield 23.4 grams (84 percent yield) of 6-chloro-7,8,9,10-tetrahydro-3-methyl-7,10-methano(1,2,4)triazolo[3,4-*a*]phthalazine. After recrystallization from benzene-hexane this product melted at 137°—138°C. Infrared spectroscopy and elemental analysis were consistent with the named structure.

The 6-chloro-7,8,9,10-tetrahydro-3-methyl-7,10-methano(1,2,4)triazolo[3,4-*a*]phthalazine (4.5 grams, 0.019 mol) was mixed with 25 milliliters of morpholine and the mixture heated at boiling under reflux for eight hours. The reaction mixture was concentrated and partitioned between methylene chloride and water. The organic layer was collected dried over sodium sulfate and evaporated to dryness to yield 4.4 grams (80.6% yield) of 7,8,9,10-tetrahydro-3-methyl-6-(4-morpholinyl)-7,10-methano(1,2,4)triazolo[3,4-*a*]-phthalazine. After recrystallization from benzene-hexane the product was found to melt at 166°—168°C. Infrared analysis and nuclear magnetic resonance analysis confirmed the assigned structure, as did elemental analysis. Calculated for $C_{15}H_{19}N_5O$: C, 63.14; H, 6.71; N, 24.55. Found: C, 63.20; H, 6.71; N, 24.32.

Examples 3—10

In a procedure similar to those of the preceding examples, the following compounds are prepared. In each case the $R_7$ and $R_8$ substituents of Formula I, taken together are 1,3-cyclopentylene, and the compounds thus correspond to the general formula

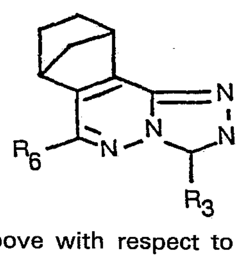

wherein $R_3$ and $R_6$ are as defined above with respect to Formula I.

| Compound | $R_3$ | $R_6$ | Melting point °C | Recrystallization solvent | Yield (percent) |
|---|---|---|---|---|---|
| 3 | H | 1-pyrrolidinyl | 189—191 | benzene-hexane | 92 |
| 4 | H | 1-piperidinyl | 122—122.5 | benzene-hexane | 85 |
| 5 | H | 4-morpholinyl | 210—203 | benzene-hexane | 67 |
| 6 | $CH_3$ | 1-pyrrolidinyl | 166—168 | benzene-hexane | 78 |
| 7 | $CH_3$ | 1-piperidinyl | 125.5—127 | benzene-hexane | 83 |
| 8* | H | 4-methyl-1-piperazinyl (dihydrochloride) | 278—280 | ethanol | 91.6 |
| 9 | $CH_3$ | 4-methyl-1-piperazinyl | 129—130 | benzene-hexane | 64.1 |
| 10* | $CH_3$ | 4-methyl-1-piperazinyl (dihydrochloride) | 271—273 | ethanol-ether | 97.4 |

*Produced by dissolving the free base in benzene and saturating the benzene solution with HCl gas to precipitate the dihydrochloride salts.

Examples 11—19

1-Chloro-4-hydrazino-5,6,7,8-tetrahydro-5,8-ethanophthalazine (2.5 grams, 0.111 mol) was mixed with 150 milliliters of formic acid. The mixture was heated at the boiling temperature under reflux for 2 hours, then evaporated to dryness. The residue was triturated with saturated aqueous sodium bicarbonate. The resulting white solid was collected, washed with water and air dried to yield 23.0 grams (88% yield) of 6-chloro-7,8,9,10-tetrahydro-7,10-ethano(1,2,4)triazolo[3,4-*a*]-phthalazine, melting at 137°—140°C. After recrystallization from a mixture of benzene and hexane the purified product melted at 140.5—141°C. Infrared spectroscopy and elemental analysis confirmed the designated structure.

In substantially the same procedure, using acetic acid instead of formic acid, 23.8 grams of 6-chloro-7,8,9,10-tetrahydro-3-methyl-7,10-ethano(1,2,4)triazolo[3,4-*a*]phthalazine was produced (86 percent yield). Melting point 178°—179°C after recrystallization from benzene-hexane.

Using 5 to 10 grams of one of the two above substituted 6-chloro-triazolopyridazines and 50 milliliters of the appropriate amine $R_6$ reactant, the following compounds were prepared. Reaction mixtures were heated at reflux for about 2 hours. Reaction mixtures were then concentrated and partitioned between water and methylene chloride. The organic layers were dried and concentrated. In each case, the $R_7$ and $R_8$ substituent is 1,4-cyclohexylene, so the compounds are nameable as 7,8,9,10-tetrahydro-3,6-substituted-7,10-ethano-1,2,4-triazolo[3,4-*a*]phthalazines, corresponding to the general formula

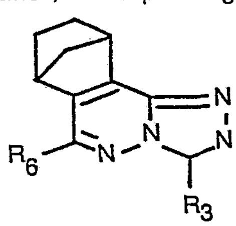

wherein $R_3$ and $R_6$ are as defined above with respect to Formula I.

| Compound | $R_3$ | $R_6$ | Melting point °C | Recrystallization solvent | Yield (percent) |
|---|---|---|---|---|---|
| 11 | H | 1-Pyrrolidinyl | 181—183 | benzene-hexane | 97 |
| 12 | H | 1-Piperidinyl | 141—143 | benzene-hexane | 85 |
| 13 | H | 4-morpholinyl | 220—221 | benzene-hexane | 71 |
| 14 | $CH_3$ | 1-pyrrolidinyl | 206—208 | benzene-hexane | 87 |
| 15 | $CH_3$ | 1-piperidinyl | 207—209 | benzene-hexane | 54 |
| 16 | $CH_3$ | 4-morpholinyl | 217—218 | benzene-hexane | 83 |
| 17 | H | 4-methyl-1-piperazinyl | 214—215 | benzene | 69 |
| 18* | H | 4-methyl-1-piperazinyl-2HCl | over 300 | ethanol-ether | 72.8 |
| 19 | $CH_3$ | 4-methyl-1-piperazinyl (free base) | 218—220 | benzene-hexane | 60 |

*Produced by dissolving 2 grams of compound 17 in 50 milliliters of benzene and saturating the solution with hydrogen chloride gas. The resulting precipitate was collected and air-dried and the structure confirmed by infrared and elemental analysis. The dihydrochloride salt 18 was dissolved in water and the solution made basic with saturated sodium bicarbonate. The mixture was extracted with methylene chloride and the extract concentrated and scratched to a solid. The structure of the resulting free base compound 17 was confirmed by infrared analysis.

Example 20

1-Chloro-4-hydrazino-5,6,7,8-tetrahydrophthalazine, also nameable as 3-chloro-4,5-tetramethylene-6-hydrazino pyridazine, (50 grams, 0.25 mol) was mixed with 250 milliliters of formic acid. The mixture was heated at the boiling temperature under reflux for 2 hours. The mixture was concentrated by evaporation under reduced pressure, and the oily residue mixed with saturated aqueous sodium bicarbonate solution. The resulting white solid was separated by filtration, washed with water and dried in air. The 6-chloro-7,8,9,10-tetrahydro(1,2,4)triazolo[3,4-*a*]phthalazine product was recrystallized from alcohol-hexane and found to melt at 124°—125°C. (Yield 42.4 grams, 81 percent). Elemental analysis confirmed the structure.

10.4 Grams (0.05 mol) of the 6-chloro-7,8,9,10-tetrahydro(1,2,4)triazolo[3,4-*a*]phthalazine were mixed with 8.53 grams (0.12 mol) pyrrolidine in 100 milliliters of ethanol. The mixture was heated at the boiling temperature under reflux for 10 hours, then concentrated by evaporation under reduced pressure until a solid residue was obtained. The solid was partitioned between methylene chloride and water; the organic layer was collected and the water layer extracted twice with methylene chloride. The methylene chloride layer and extracts were combined, dried over anhydrous sodium sulfate, filtered and evaporated under reduced pressure. The resulting colorless solid 6-(1-pyrrolidinyl)-7,8,9,10-tetrahydro(1,2,4)triazolo[3,4-*a*]-phthalazine product was crystallized from ethanol and found to melt at 194—195.5°C. 9 Grams of product were obtained, a 74 percent yield from this step. Elemental analysis confirmed the structure (Calculated for $C_{13}H_{17}N_5$: C, 64.17; H, 7.04; N, 28.79. Found: C, 64.4; H, 7.01; N, 28.83.) The product is also named as 6-pyrrodilidino-7,8-tetramethylene-*s*-triazolo[4,3-*b*]pyridazine.

Example 21

In a similar procedure, 6-piperidino-7,8-tetramethylene-*s*-triazolo[3,4-*b*]pyridazine, also named as 6-piperidino-7,8,9,10-tetrahydro(1,2,4)triazolo[3,4-*a*]phthalazine, melting at 134.5—135.5°C was prepared.

Example 22

In a procedure similar to those of the preceding examples, 6-chloro-7,8,9,10-tetrahydro(1,2,4)triazolo[3,4-*a*]phthalazine (prepared as in Example 20) (10 grams, 0.048 mol) and 12.2 grams (0.122 mol) of N-methylpiperazine in 100 milliliters of ethanol were heated at reflux for 20 hours. Thin layer chromatography (TLC) showed the presence of unreacted phthalazine. The ethanol was evaporated, 50 milliliters of N-methylpiperazine were added and the mixture heated at reflux for 2 hours, after which TLC indicated the reaction to be complete. The mixture was concentrated, and partitioned between methylene chloride and water, and the residue from evaporation of the methylene chloride was recrystallized twice from a mixture of benzene and hexane, then once from hexane. The 6-(4-methyl-1-piperazinyl)-7,8-tetramethylene(1,2,4)triazolo[4,3-*b*]pyridazine product was found to melt at

160—162°C. 12.45 Grams of the product were obtained (95 percent yield). The structure was confirmed by nuclear magnetic resonance analysis and infrared spectroscopy. (Calculated: C, 61.74; H, 7.40; N, 30.86. Found: C, 61.90; H, 7.35; N, 30.59). Nuclear magnetic resonance ($CDCl_3$); δ 9.84 (s, 1, CH); 3.4—3.0 (m, 6); 2.8—2.5 (m, 6); 2.38 (s, 3, $CH_3$); 2.1—1.7 (m, 4, $CH_2CH_2CH_2CH_2$). Melting point of monohydrochloride salt 265°—266°C.

In similar procedures, the following are prepared:

6-Morpholino-7,8-tetramethylene-*s*-triazolo[4,3-*b*]pyridazine, also named as 6-morpholino-7,8,9,10-tetrahydro(1,2,4)triazolo[3,4-*a*]phthalazine, melting at 194°—196°C;

6-(2-Methylpiperidino)-7,8-tetramethylene-*s*-triazolo-[4,3-*b*]pyridazine, melting at 84°—86°, molecular weight 299.41.

6-Piperidino-3-methyl-7,8-tetramethylene-*s*-triazolo[4,3-*b*]pyridazine, melting at 144—144.5°C;

6-Morpholino-3-methyl-7,8-tetramethylene-*s*-triazolo[4,3-*b*]pyridazine, melting at 189—190°C.

Example 23

6-Chloro-7,8,9,10-tetrahydro(1,2,4)triazolo[3,4-*a*]phthalazine (7.2 grams; 0.0345 mole) was mixed with 20 milliliters of hexamethyleneimine and 100 milliliters of methanol. The mixture was heated at the boiling temperature under reflux for 30 hours, cooled and partitioned between methylene chloride and water. The methylene chloride layer was dried and evaporated to dryness. The residue was recrystallized from benzene-hexane to give the 6-(hexahydro-1H-azepin-1-yl)-7,8-tetramethylene(1,2,4)triazolo[4,3-*b*]pyridazine as colorless crystals, melting at 109°C. Elemental analysis: C, H, N calculated: 66.39; 7.80; 25.81; C, H, N found: 66.04; 7.42; 25.76. The above reaction was carried out without the methanol reaction medium, and the product was found to melt at 108°—109°C.

Example 24

15.65 Grams of 6-chloro-7,8-tetramethylene(1,2,4)triazolo[4,3-*b*]pyridazine and 35 milliliters of 2-methylpyrrolidine were mixed and heated under reflux for two hours. The reaction mixture was evaporated to dryness, and partitioned between methylene chloride and water. The methylene chloride layer was dried and evaporated to leave 12.5 grams (64.8% yield) of 6-(2-methylpyrrolidinyl)-7,8-tetramethylene(1,2,4)triazolo[4,3-*b*]pyridazine. The product was recrystallized from benzene-hexane and found to melt at 146°—147°C. C, H, N calculated: 65.34; 7.44; 27.22. C, H, N found: 65.5; 7.51; 27.36.

Examples 25—31

In procedures similar to those of Examples 23 and 24, the following 6-substituted-7,8-tetramethylene-*s*-triazolo[4,3-*b*]pyridazines were prepared. The compounds are identified below by the $R_6$ substituents; $R_7$ and $R_8$ being tetramethylene and $R_3$ being hydrogen.

| Example | $R_6$ | Yield (percent) | Melting point °C |
|---|---|---|---|
| 25 | methylamino | 86 | 155—156 |
| 26 | dimethylamino | 71.7 | 153—154 |
| 27 | 3-methylpiperidino | 100 | 99—102 |
| 28 | 4-methylpiperidino | 100 | 152.5—154 |
| 29 | hexahydro-4-methyl-1H-1,4-diazepin-1-yl | 63.3 | 135—136 |
| 30 | dihydrochloride salt of 29 | | 249—251 |
| 31 | ethylamino | — | 250—251 |

Examples 32—38

In a similar procedure, the following compounds were prepared, in which $R_3$ is methyl, $R_7$ and $R_8$ are tetramethylene, and $R_6$ is identified below.

| Example | $R_6$ | Yield (percent) | Melting point °C |
|---|---|---|---|
| 32 | methylamino | 78.1 | 279—280 |
| 33 | dimethylamino | 72.6 | 128—129 |
| 34 | 2-methyl-1-pyrrolidinyl | 50 | 142 |
| 35 | 3-methylpiperidino | 61 | 152—153 |
| 36 | 4-methylpiperidino | 81.7 | 143—144 |
| 37 | 4-methyl-1-piperazinyl | 66.8 | 183 |
| 38 | hexahydro-4-methyl-1H-1,4-diazepin-1-yl | 56.6 | 114—115 |
| | dihydrochloride | 56.9 | 240—241 |

Examples 39—47

A. A solution of 98.5 grams (0.496 mole) of 1-chloro-4-hydrazino-5,6,7,8-tetrahydrophthalazine in 300 milliliters of propionic acid was heated at reflux for 18 hours, then concentrated by evaporation to half the original volume. The solution was diluted with aqueous sodium carbonate until neutral (pH 7). The resulting precipitate was collected, air-dried at ambient temperature, then dried in a drying oven to produce 105 grams (0.444 mol) of 6-chloro-3-ethyl-7,8,9,10-tetrahydro-1,2,4-triazolo-[3,4-*a*]phthalazine, melting at 93°—94°C (89.6% yield). Calculated for $C_{11}H_{13}ClN_4$: C, 55.81; H, 5.53; N, 23.67. Found: C, 56.00; H, 5.63; N, 23.87.

B. In a procedure similar to the foregoing examples, the 6-chlorotetramethylenetriazolopyridazine intermediate product was reacted with excess $R_6$ amine (more than two equivalents) at reflux. Reaction progress was monitored by thin layer chromatography on silica gel, developed with 9:1-chloroform:methanol, where the 6-chloro intermediate appeared as a fluorescent spot and the product was nonfluorescent. After reaction was complete, the reaction mixtures were concentrated, and the residue partitioned between water and methylene chloride. The organic layer was dried with sodium sulfate, concentrated and the residue recrystallized from benzene-hexane. Salts were formed by dissolving the base in acetone and adding ethereal acid (hydrogen chloride). Using the above 6-chloro intermediate, the following were prepared, in which $R_3$ is ethyl, $R_7$ and $R_8$ are tetramethylene, and $R_6$ is identified below.

| Example | $R_6$ | Yield (percent) | Melting point °C |
|---|---|---|---|
| 39 | hexahydro-4-methyl-1H-1,4-diazepin-1-yl | 44 | 95—96 |
| 40 | dihydrochloride of 39* | 53 | 225—228 |
| 41 | hexahydroazepin-1-yl | 28.2 | 104 |
| 42 | morpholino | 62.0 | 170—171 |
| 43 | piperidino | 57.8 | 135—136 |
| 44 | 2-methylpyrrolidino | 55.2 | 152—153 |
| 45 | pyrrolidino | 59.8 | 118—119 |
| 46 | 4-methyl-1-piperazinyl | 67.8 | 172—173 |
| 47 | dihydrochloride of 46** | 86.7 | 250—252 |

*Recrystallized from methanol-acetone
**Recrystallized from isopropanol

Examples 48—50

A. In a procedure similar to that of Example 39A, 98.5 grams of 1-chloro-4-hydrazino-5,6,7,8-tetrahydrophthalazine were heated at reflux in 300 milliliters of isobutyric acid. After 18 hours, excess isobutyric acid was distilled off and the residue neutralized with aqueous sodium bicarbonate. The

resulting solid 6-chloro-7,8,9,10-tetrahydro-3-(1-methylethyl)-1,2,4-triazolo[3,4-*a*]-phthalazine was collected and air-dried to yield 100 grams (80.4 percent yield). After recrystallization from isopropanol the yield was 56.2 grams, melting at 67—68°C.

B. Using the above 6-chloro intermediate product, and the procedure described in Example 39B and the preceding Examples, the following compounds were prepared, in which $R_3$ is isopropyl, $R_7$ and $R_8$ are tetramethylene, and $R_6$ is identified below:

| Example | $R_6$ | Yield (percent) | Melting point °C |
|---|---|---|---|
| 48 | hexahydro-4-methyl-1H-1,4-diazepin-1-yl | 55.9 | 62—63 |
| 49 | 2-methyl-pyrrolidino | 44.5 | 129—130 |
| 50 | pyrrolidino | 32.6 | 119—121 |

Example 51

One gram (0.49 mole) of 3,6-dichloro-4,5-tetramethylenepyridazine, 78.3 grams (0.738 mole) sodium carbonate, and 500 milliliters of diglyme (diethylene glycol dimethyl ether) were mixed. 46.1 Grams (0.54 mole) of 2-methylpyrrolidine was added slowly with stirring, and the mixture was heated at reflux (134.5°C) for 72 hours, then cooled to 95°C. 400 Milliliters deionized water was added, and the mixture was cooled to about 25°C. The resulting crystals were separated by filtration, washed with water and dried under reduced pressure to yield 104.0 grams of 3-chloro-6-(2-methyl-1-pyrrolidinyl)-4,5-tetramethylenepyridazine, melting at 134—136°C.

210 Grams (0.834 mole) of 3-chloro-6-(2-methyl-1-pyrrolidinyl)-4,5-tetramethylenepyridazine, 216 grams (2.92 moles) ethyl formate and 1260 milliliters of ethylene glycol ethyl ether were mixed. Over a 10 minute period, 146.2 grams (2.92 moles) of hydrazine hydrate was added, to form formyl hydrazine *in situ*. During the addition, the temperature increased from 25°C to 35°C. The mixture was heated at 100°C for about 30 minutes, during which time 100 milliliters of ethanol, water and the reaction medium were collected in a Dean Stark trap. The mixture was then heated to reflux at 120°C for 24 hours, cooled to 95°C, and diluted slowly with about 5 liters of deionized water. The solution was cooled and seeded with product crystals at 60°C, then cooled to 25°C. The crystalline product was collected by filtration, washed with four 500 milliliter portions of deionized water and dried under reduced pressure for about 18 hours at 95°C. 144.3 Grams (67.2% yield) of 6-(2-methyl-1-pyrrolidinyl)-7,8-tetramethylene-1,2,4-triazolo[4,3-*b*]pyridazine were obtained as a crystalline solid, melting at 144—145°C.

Example 52

Bronchodilator activity of representative triazolopyridazine compounds of the invention is examined in the Konzett-Rossler guinea pig preparation according to accepted procedures. See Konzett and Rossler; Arch. f. Exp. Path. u. Pharmakol. *195*: 71—74 (1940); Rosenthale and Dervinis, Arch. Int. Pharmacodyn. *172*: 9194 (1968). In this procedure an anesthetized guinea pig is artificially respired with a fixed volume of air. The volume of air is selected to slightly exceed the capacity of the guinea pig's lungs, and the excess air or "overflow" is measured. Test compounds are evaluated by administering a test compound intravenously 2 minutes prior to administration of an agonist compound (histamine, serotonin or acetylcholine) following 3 previous agonist doses resulting in relatively uniform (±10 percent) bronchoconstriction. When the bronchospasm resulting from administration of the agonist compound occurs, the animal's lungs can receive less air, and hence the "overflow" is measurably increased. When a test compound blocks the bronchoconstriction induced by administration of the agonist compound, the results can be expressed quantitatively as a percent blockade. This is calculated by dividing the "overflow" agonist response measured after administration of the test compound by the average of the 3 preceding agonist responses, multiplying by 100 and subtracting this value from 100 percent. The results can also be expressed in comparison to a known bronchodilator, such as aminophylline. In such procedure, comparative results are expressed as "percent aminophylline", calculated by expressing the percentage blockade produced by a test compound as a percentage of the average percent blockade produced by dosages of aminophylline administered to the same test animal employed for the test compound with the aminophylline observations preceding and following the evaluation of the test compound in that animal.

In representative operations with triazolopyridazine compounds of the invention, administered intravenously at a dosage rate of 3 milligrams of test compound per kilogram of animal body weight (except as otherwise indicated) and using aminophylline at a dosage rate of 10 milligrams per kilogram for comparison and histamine as the agonist, representative compounds gave excellent results in terms of percent blockade of histamine and percent of aminophylline.

The compound 6-pyrrolidino-7,8-tetramethylene-*s*-triazolo[4,3-*b*]pyridazine and the compound 6-morpholino-7,8-tetramethylene-*s*-triazolo[4,3-*b*]pyridazine administered as an aqueous solution gave 100 and 75 percent blockade of histamine, respectively, amounting to 105 and 113 percent of aminophylline activity.

Example 53

Audiogenic convulsive side effects

Certain xanthine compounds, such as the known bronchodilator aminophylline, have central nervous system stimulant side effects which are difficult to detect in animal models which are satisfactory for evaluating other compounds. The interaction of sound with the convulsive threshold of drugs is a known phenomenon which can be used to evaluate such side effects. See, for example, Schlesinger et al., Life Science *4*, 2345—2351 (1965), *7*, 437—447 (1968) and *9* (I) 721—729 (1970); Buckholtz, Pharmacol. Biochem. and Behavior *3*, 65—68 (1975); and U.S. Patent 4,136,182. In a procedure for pharmacological evaluation, the lowering of the convulsive threshold, or the lowering of the $LD_{50}$, by sound can be studied in mice.

In the test operations, mice are administered a test compound by intraperitoneal injection at various dosages, and the number of mice showing tonic convulsions and the number of fatalities occurring within 30 minutes is recorded. The $ED_{50}$ for tonic convulsions, and the 30 minute $LD_{50}$ are then determined. These operations are carried out in standard laboratory cages with mice that have become acclimated to the laboratory.

The surviving mice are then also exposed to sound about 30 minutes after dosing. The sound exposure is carried out by placing the mice in a sound insulated cage with a bell which emits 120 decibels of sound, and activating the bell for two minutes. The number of tonic convulsions and fatalities are then recorded to determine the $ED_{50}$ and $LD_{50}$ in the presence of the sound challenge.

In a series of similar experiments, the ratio of the 30 minute $LD_{50}$ without sound to the sound-induced $LD_{50}$ for aminophylline, theophylline and caffeine has been found to be greater than 3 for all three compounds, while strychnine exhibited no significant change in toxicity with sound. U.S. Patent 4,136,182.

Various triazolopyridazine compounds have been found to exhibit increased toxicity and lowered convulsive thresholds in such procedures, similar to aminophylline. Other triazolopyridazines which have bronchodilator activity exhibit much less toxic potential for audiogenic seizures. For example, the ratio of $LD_{50}$ without sound to $LD_{50}$ with sound for 6-morpholino-7,8-tetramethylene-*s*-triazolo[4,3-*b*]pyridazine was found to be 3.8 while the ratio for 6-morpholino-3-methyl-7,8-tetramethylene-*s*-triazolo[4,3-*b*]pyridazine was found to be about 1.18. Surprisingly, some triazolopyridazines have been found to exhibit a significant lowering of audiogenic convulsive threshold without an associated increase in sound-induced deaths, as indicated by a low $ED_{50}$ for audiogenic tonic convulsions in comparison to the $LD_{50}$ with sound.

The following table illustrates the bronchodilator activity of representative compounds of the invention and their acute toxicity and audiogenic convulsive properties.

| Compound | | Histamine blockade | 30 min $LD_{50}$ | Sound 1 $LD_{50}$ | Audiogenic convulsant $ED_{50}$ |
|---|---|---|---|---|---|
| Compounds in which $R_7$, $R_8$ are tetramethylene | | | | | |
| $R_3$ | $R_6$ | | | | |
| H | piperidino | 72 | 298 | 298 | 283 |
| H | N-methyl-1-piperazinyl | 91 | 147 | 134 | 134 |
| $CH_3$ | morpholino | 61 | 149 | 126 | 90 |
| $CH_3$ | piperidino | 38 | 139 | 139 | 125 |
| H | pyrrolidino | 86 | 191 | 170 | 150 |

| Compound | | Histamine blockade | 30 min $LD_{50}$ | Sound 1 $LD_{50}$ | Audiogenic convulsant $ED_{50}$ |
|---|---|---|---|---|---|
| Compounds in which $R_7$, $R_8$ are 1,3-cyclopentylene | | | | | |
| $R_3$ | $R_6$ | | | | |
| $CH_3$ | N-methyl-1-piperazinyl | 97 | 147 | 147 | 147 |
| H | N-methyl-1-piperazinyl | 92 | 175 | 147 | 147 |
| $CH_3$ | pyrrolidino | 95 | 85 | 85 | 85 |
| H | pyrrolidino | 87 | 136 | 100 | 93 |
| H | piperidino | 84 | 108 | 98 | 90 |
| $CH_3$ | morpholino | 76 | 86 | 74 | 61 |
| Compounds in which $R_7$, $R_8$ are 1,4-cyclohexylene | | | | | |
| $R_3$ | $R_6$ | | | | |
| $CH_3$ | pyrrolidino | 55 | 141 | 141 | 141 |
| H | pyrrolidino | 66 | 136 | 117 | 117 |
| $CH_3$ | N-methyl-1-piperazinyl | 90 | 147 | 147 | 147 |
| H | N-methyl-1-piperazinyl | 82 | 147 | 147 | 113 |
| H | morpholino | 58 | 118 | 85 | 62 |

Example 54

Histamine aerosol exposure

In another procedure, test compounds were administered to guinea pigs by intraperitoneal injection and the guinea pigs were challenged two hours later by exposure to a histamine aerosol. Untreated animals collapse when exposed to the histamine aerosol. In these operations, the animals were observed, and an $ED_{50}$ was calculated as the dosage at which fifty percent of the animals displayed a collapse time greater than the mean collapse time plus two standard deviation units observed with control animals treated with the injection vehicle alone. The $ED_{50}$'s of representative compounds are set out in the following table.

| Compound of Example No. | ED$_{50}$(mg/kg i.p.) |
|---|---|
| 23 | 5.0 |
| 24 | 1.6 |
| 25 | 4.0 |
| 26 | 2.5 |
| 27 | 1.8 |
| 28 | 1.3 |
| 32 | 2.1 |
| 33 | 2.2 |
| 38 (dihydrochloride) | 23.8 |
| 39 | 5.0 |
| 40 | < |
| 41 | (estimated) 4.95 |
| 45 | <2.5 |
| 47 | 36.4 |
| 49 | (estimated) 7.9 |
| 50 | (estimated) 5.1 |

**Claims for the Contracting States: BE CH DE FR IT LI LU NL SE**

1. A compound corresponding to the formula

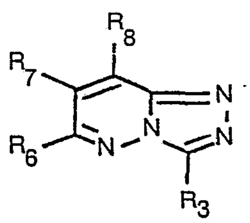

wherein $R_3$ represents hydrogen or $(C_{1-4})$alkyl; $R_6$ represents amino, $(C_{1-4})$alkylamino, di-$(C_{1-4})$alkylamino, heterocyclic amino or $(C_{1-4})$alkyl substituted heterocyclic amino, wherein the heterocyclic moiety forms a 5-, 6- or 7-membered ring, having one or two ring nitrogen atoms and zero or one ring sulfur or oxygen atom; $R_7$ and $R_8$ taken together represent polymethylene or substituted polymethylene of 4 methylene units substituted by $(C_{1-4})$alkyl, or by methano or ethano bridges; and the pharmacologically acceptable salts of said compound.

2. A compound of claim 1 wherein the compound corresponds to the formula

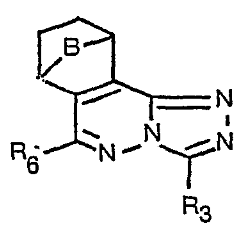

wherein B represents methylene or ethylene.

3. A compound of claim 1 wherein $R_7$ and $R_8$ are tetramethylene, $R_3$ is hydrogen and $R_6$ is 2-methyl-1-pyrrolidinyl.

4. A compound as in claim 1 wherein $R_3$, $R_7$, and $R_8$ are as defined in claim 1 and $R_6$ represents a heterocyclic amino or $(C_{1-4})$alkyl substituted heterocyclic amino wherein the heterocyclic moiety forms a 5-, 6- or 7-membered ring; and the pharmacologically acceptable salts of said compound.

5. Compound of claim 4 wherein $R_7$ and $R_8$ are tetramethylene, $R_3$ is hydrogen and $R_6$ is hexahydro-1H-azepin-1-yl.

6. Compound of claim 4 wherein $R_7$ and $R_8$ are tetramethylene, $R_3$ is hydrogen and $R_6$ is pyrrolidino.

7. Compound of claim 4 wherein $R_7$ and $R_8$ are tetramethylene, $R_3$ is hydrogen and $R_6$ is piperidino.

8. A compound as in claim 1 which is 6-(hexahydro-4-methyl-1H-1,4-diazepin-1-yl)-3-methyl-7,8-tetramethylene-*s*-triazolo[4,3-*b*]pyridine.

9. A bronchodilator composition comprising an effective amount of a compound of claim 1 in admixture with a pharmaceutically-acceptable carrier.

10. A process for making a compound according to claim 1 which comprises reacting a 3,6-dihalo-4,5-substituted pyridazine having the formula

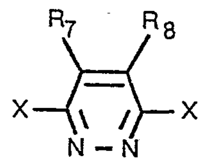

wherein $R_7$ and $R_8$ are as above identified, and X stands for fluoro, chloro or bromo, with an amine corresponding to the above identified $R_6$ in an inert solvent having boiling point of 40°C to 200°C in the presence of an inorganic base or a non-nucleophilic amine, or an excess of the $R_6$ amine as hydrogen halide acceptor, at a temperature of 25°C to 150°C, to form a 3-halopyridazine, which is then reacted with a compound having the formula

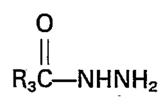

wherein $R_3$ is as above identified, in an inert liquid medium at a temperature of 50°C to 200°C; or a compound of the formula

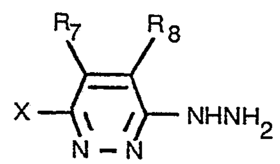

wherein $R_7$ and $R_8$ are as above identified, and X stands for fluoro, chloro or bromo, is reacted at a temperature of 40°C to 150°C with a compound of the formula

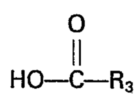

wherein $R_3$ is as above identified, in an excess of acid optionally diluted with an inert solvent having a boiling point of 40°C to 200°C, and the resulting 6-halotriazolopyridazine is then reacted with an amine corresponding to the above identified $R_6$, at a temperature of 75°C to 160°C in the presence of an inorganic base or a non-nucleophilic amine or an excess of the $R_6$ amine as hydrogen halide acceptor in an inert solvent having boiling point of 75°C to 200°C or with an excess of the same $R_6$ amine as the reaction medium at the boiling temperature.

11. A process as in claim 10 wherein the inert solvent is an inert organic solvent.

12. A process as in claim 10 wherein the inert solvent is a hydroxylic solvent.

13. A process as in claim 10 wherein an excess of the same $R_6$ amine is employed as the reaction medium at the boiling temperature.

**Claims for the Contracting State: AT**

1. A process for making a compound having the formula:

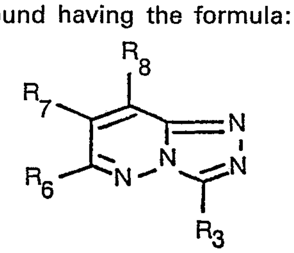

wherein $R_3$ represents hydrogen or $(C_{1-4})$alkyl; $R_6$ represents amino, $(C_{1-4})$alkylamino, di-$(C_{1-4})$alkylamino, heterocyclic amino or $(C_{1-4})$alkyl substituted heterocyclic amino, wherein the heterocyclic moiety forms a 5-, 6- or 7-membered ring having one or two ring nitrogen atoms and zero or one ring sulfur or oxygen atom; $R_7$ and $R_8$ taken together represent polymethylene or substituted polymethylene of 4 methylene units substituted by $(C_{1-4})$alkyl or by methano or ethano bridges; and the pharmacologically acceptable salts of said compound; which comprises reacting a 3,6-dihalo-4,5-substituted pyridazine having the formula

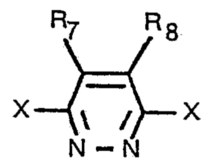

wherein $R_7$ and $R_8$ are as above identified and X stands for fluoro, chloro or bromo, with an amine corresponding to the above identified $R_6$, in an inert solvent having boiling point 40°C to 200°C and in the presence of an inorganic base, or a non-nucleophilic amine, or an excess of the $R_6$ amine as hydrogen halide acceptor, at a temperature of 25° to 150°C, to form a 3-halopyridazine, which is then reacted with a compound having the formula

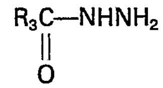

wherein $R_3$ is as above identified, in an inert liquid medium at a temperature of 50°C to 200°C; or a compound of the formula

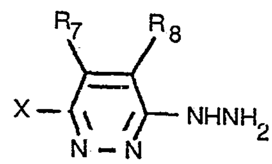

wherein $R_7$ and $R_8$ are as above identified and X stands for fluoro, chloro or bromo, is reacted at a temperature of 40°C to 150°C with a compound of the formula

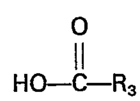

wherein $R_3$ is as above identified, in an excess of acid optionally diluted with an inert solvent having boiling point of 40°C to 200°C, and the resulting 6-halotriazolopyridazine is then reacted with an amine corresponding to the above identified $R_6$, at a temperature of 75°C to 160°C in the presence of an inorganic base or a non-nucleophilic amine or an excess of the $R_6$ amine as hydrogen halide acceptor in an inert solvent having boiling point 75°C to 200°C or with an excess of the same $R_6$ amine as the reaction medium at the boiling temperature.

2. A process as in claim 1 wherein the inert solvent is an inert organic solvent.

3. A process as in claim 1 wherein the inert solvent is a hydroxylic solvent.

4. A process as in claim 1 wherein an excess of the same $R_6$ amine is employed as reaction medium at the boiling temperature.

5. Process as in claim 1 for preparing a compound of formula I wherein $R_6$ represents heterocyclic amino or $(C_1—C_4)$alkyl substituted heterocyclic amino wherein the heterocyclic moiety forms a 5-, 6- or 7-membered ring.

6. Process of claim 5 wherein $R_6$ is 2-methyl-1-pyrrolydinyl.

7. Process of claim 6 wherein $R_7$ and $R_8$ are tetramethylene and $R_3$ is hydrogen.

8. Process of claim 5 wherein $R_7$ and $R_8$ are tetramethylene, $R_3$ is hydrogen and $R_6$ is pyrrolidino.

9. Process of claim 5 wherein $R_7$ and $R_8$ are tetramethylene, $R_3$ is hydrogen and $R_6$ is hexahydro-1H-azepin-1-yl.

10. Process of claim 5 wherein $R_7$ and $R_8$ are tetramethylene, $R_3$ is hydrogen and $R_6$ is piperidino.

11. A process as in claim 1 for preparing 6-(hexahydro-4-methyl-1H-1,4-diazepin-1-yl)-3-methyl-7,8-tetramethylene-*s*-triazolo[4,3-*b*]pyridine.

**Patentansprüche für die Vertragsstaaten: BE CH DE FR IT LI LU NL SE**

1. Verbindung der allgemeinen Formel

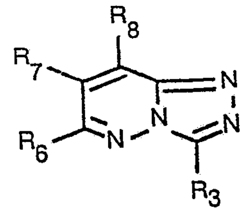

in der $R_3$ ein Wasserstoffatom oder ein $(C_{1-4})$-Alkylrest ist; $R_6$ eine Amino-, $(C_{1-4})$-Alkylamino-, Di-$(C_{1-4})$-alkylamino-, eine heterocyclische Amino- oder eine $(C_{1-4})$-Alkyl-substituierte heterocyclische Aminogruppe ist, in der der heterocyclische Rest einen 5-, 6- oder 7- Ring bildet, der ein oder zwei Stickstoffatome und kein oder ein Schwefel- oder Sauerstoffatom trägt; und in der $R_7$ und $R_8$ eine Polymethylengruppe oder eine substituierte Polymethylengruppe mit vier Methyleneinheiten sind, die durch einen $(C_{1-4})$-Alkylrest oder durch Methan- oder Äthanbrücken substituiert sind; sowie die pharmazeutisch verträglichen Salze dieser Verbindungen.

2. Verbindung nach Anspruch 1, die die folgende Formel hat:

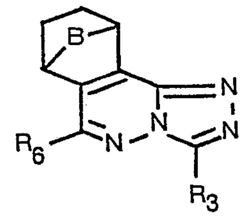

in der B ein Methylen- oder Äthylenrest ist.

3. Verbindung nach Anspruch 1, in der $R_7$ und $R_8$ Tetramethylgruppen sind, $R_3$ ein Wasserstoffatom und $R_6$ eine 2-Methyl-1-pyrrolidinylgruppe ist.

4. Verbindung nach Anspruch 1, in der $R_3$, $R_7$ und $R_8$ die in Anspruch 1 angegebene Bedeutung haben und in der $R_6$ eine heterocyclische Amino- oder $(C_{1-4})$-Alkyl-substituierte heterocyclische Aminogruppe ist, in der der heterocyclische Rest einen 5-, 6- oder 7-Ring bildet; und die pharmazeutisch verträglichen Salze dieser Verbindungen.

5. Verbindung nach Anspruch 4, in der $R_7$ und $R_8$ Tetramethylengruppen sind und in der $R_3$ ein Wasserstoffatom und $R_6$ eine Hexahydro-1H-azepin-1-yl-Gruppe ist.

6. Verbindung nach Anspruch 4, in der $R_7$ und $R_8$ Tetramethylengruppen sind und in der $R_3$ ein Wasserstoffatom und $R_6$ eine Pyrrolidingruppe ist.

7. Verbindung nach Anspruch 4, in der $R_7$ und $R_8$ Tetramethylengruppe sind und in der $R_3$ ein Wasserstoffatom und $R_6$ eine Piperidingruppe ist.

8. 6-(Hexahydro-4-methyl-1H-1,4-diazepin-1-yl)-3-methyl-7,8-tetramethylen-*s*-triazolo[4,2-*b*]pyridin nach Anspruch 1.

9. Bronchodilatorisches Mittel enthaltend eine wirksame Menge einer Verbindung nach Anspruch 1 und ein pharmazeutisch verträgliches Trägermaterial.

10. Verfahren zur Herstellung einer Verbindung nach Anspruch 1, bei dem ein 3,6-Dihalo-4,5-substituiertes Pyridazin der allgemeinen Formel

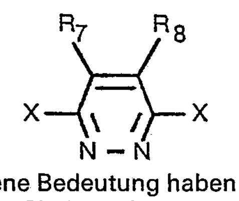

in der $R_7$ und $R_8$ die vorstehend angegebene Bedeutung haben und X ein Fluor-, Chlor- oder Bromatom ist, in einem inerten Lösungsmittel mit einem Siedepunkt von 40 bis 200°C mit einem wie vorstehend als $R_6$ definierten Amin in Gegenwart einer anorganischen Base oder eines nicht-nucleophilen Amins oder eines Überschusses des Amins $R_6$ als Halogenwasserstoffakzeptor bei einer Temperatur von 25 bis 150°C umgesetzt wird, wobei sich ein 3-Halogenpyridazin bildet, das anschließend mit einer Verbindung der allgemeinen Formel

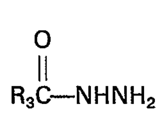

in der $R_3$ die vorstehend angegebene Bedeutung hat, bei einer Temperatur von 50 bis 200°C in einem inerten flüssigen Medium umgesetzt wird; oder bei dem eine Verbindung der allgemeinen Formel

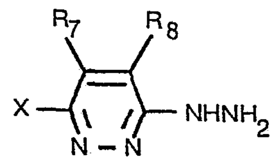

in der $R_7$ und $R_8$ die vorstehend angegebene Bedeutung haben und X ein Fluor- Chlor- oder Bromatom ist, bei einer Temperatur von 40 bis 150°C mit einer Verbindung der allgemeinen Formel

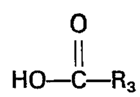

in der $R_3$ die vorstehend angegebene Bedeutung hat, mit einem Überschuß von gegebenenfalls mit einem inerten Lösungsmittel mit einem Siedepunkt von 40 bis 200°C verdünnter Säure umgesetzt wird und das dabei entstandene 6-Halogentriazolpyridazin anschließend mit einem vorstehend als $R_6$ definierten Amin bei einer Temperatur von 75 bis 160°C in Gegenwart einer anorganischen Base oder eines nicht-nucleophilen Amins oder eines Überschusses des Amins $R_6$ als Halogenwasserstoffakzeptor in einem inerten Lösungsmittel mit einem Siedepunkt von 75 bis 200°C oder mit einem Überschuß des genannten Amins $R_6$ als Reaktionsmedium bei der Siedetemperatur umgesetzt wird.

11. Verfahren nach Anspruch 10, in dem das inerte Lösungsmittel ein inertes organisches Lösungsmittel ist.

12. Verfahren nach Anspruch 10, in dem das inerte Lösungsmittel ein hydroxylisches Lösungsmittel ist.

13. Verfahren nach Anspruch 10, bei dem ein Überschuß des genannten Amins $R_6$ als Reaktionsmedium bei der Siedetemperatur verwendet wird.

**Patentansprüche für den Vertragsstaat: AT**

1. Verfahren zur Herstellung einer Verbindung der allgemeinen Formel

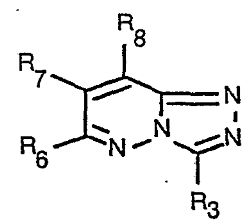

in der $R_3$ ein Wasserstoffatom oder ein $(C_{1-4})$-Alkylrest ist; $R_6$ eine Amino-, $(C_{1-4})$-Alkylamino-, Di-$(C_{1-4})$-alkylamino-, eine heterocyclische Amino- oder eine $(C_{1-4})$-Alkyl-substituierte heterocyclische Aminogruppe ist, in der der heterocyclische Rest einen 5-, 6- oder 7- Ring bildet, der ein oder zwei Stickstoffatome und kein oder ein Schwefel- oder Sauerstoffatom aufweist; in der $R_7$ und $R_8$ eine Polymethylen- oder eine substituierte Polymethylengruppe mit vier Methyleneinheiten sind, die durch einen $(C_{1-4})$-Alkylrest oder durch Methan- oder Äthanbrücken substituiert sind; und die pharmazeutisch verträglichen Salze dieser Verbindungen, bei dem ein 3,6-Dihalo-4,5-substituiertes Pyridazin der allgemeinen Formel

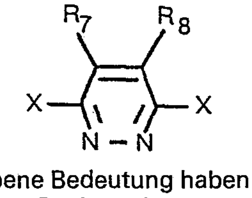

in der $R_7$ und $R_8$ die vorstehend angegebene Bedeutung haben und X ein Fluor-, Chlor- oder Bromatom ist, in einem inerten Lösungsmittel mit einem Siedepunkt von 40 bis 200°C mit einem wie vorstehend als $R_6$ definierten Amin in Gegenwart einer anorganischen Base oder eines nicht-nucleophilen Amins oder eines Überschusses des Amins $R_6$ als Halogenwasserstoffakzeptor bei einer Temperatur von 25 bis 150°C umgesetzt wird, wobei sich ein 3-Halogenpyridazin bildet, das anschließend mit einer Verbindung der allgemeinen Formel

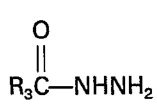

in der $R_3$ die vorstehend angegebene Bedeutung hat, bei einer Temperatur von 50 bis 200°C in einem inerten flüssigen Medium umgesetzt wird; oder bei dem eine Verbindung der allgemeinen Formel

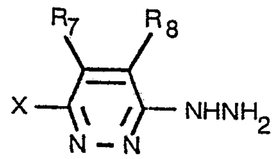

in der $R_7$ und $R_8$ die vorstehend angegebene Bedeutung haben und X ein Fluor-, Chlor- oder Bromatom ist, bei einer Temperatur von 40 bis 150°C mit einer Verbindung der allgemeinen Formel

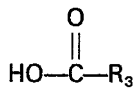

in der $R_3$ die vorstehend angegebene Bedeutung hat, mit einem Überschuß von gegebenenfalls mit einem inerten Lösungsmittel mit einem Siedepunkt von 40 bis 200°C verdünnter Säure umgesetzt wird und das dabei entstandene 6-Halogentriazolpyridazin anschließend mit einem vorstehend als $R_6$ definierten Amin bei einer Temperatur von 75 bis 160°C in Gegenwart einer anorganischen Base oder eines nicht-nucleophilen Amins oder eines Überschusses des Amins $R_6$ als Halogenwasserstoffakzeptor in einem inerten Lösungsmittel mit einem Siedepunkt von 75 bis 200°C oder mit einem Überschuß des genannten Amins $R_6$ als Reaktionsmedium bei der Siedetemperatur umgesetzt wird.

2. Verfahren nach Anspruch 1, wobei das inerte Lösungsmittel ein inertes organisches Lösungsmittel ist.

3. Verfahren nach Anspruch 1, wobei das inerte Lösungsmittel ein hydroxylisches Lösungsmittel ist.

4. Verfahren nach Anspruch 1, wobei ein Überschuß des genannten Amins $R_6$ als Reaktionsmedium bei der Siedetemperatur verwendet wird.

5. Verfahren nach Anspruch 1 zur Herstellung einer Verbindung der allgemeinen Formel I, wobei $R_6$ eine heterocyclische Aminogruppe oder eine ($C_1$—$C_4$)-Alkyl-substituierte heterocyclische Aminogruppe ist, in der der heterocyclische Rest einen 5-, 6- oder 7-Ring bildet.

6. Verfahren nach Anspruch 5, in dem $R_6$ 2-Methyl-1-pyrrolidinyl ist.

7. Verfahren nach Anspruch 6, in dem $R_7$ und $R_8$ eine Tetramethylengruppe sind und in dem $R_3$ ein Wasserstoffatom ist.

8. Verfahren nach Anspruch 5, in dem $R_7$ und $R_8$ eine Tetramethylengruppe sind und in dem $R_3$ ein Wasserstoffatom und $R_6$ eine Pyrrolidingruppe ist.

9. Verfahren nach Anspruch 5, in dem $R_7$ und $R_8$ eine Tetramethylengruppe sind und $R_3$ ein Wasserstoffatom und $R_6$ eine Hexahydro-1H-azepin-1-yl-Gruppe ist.

10. Verfahren nach Anspruch 5, in dem $R_7$ und $R_8$ eine Tetramethylengruppe sind und $R_3$ ein Wasserstoffatom und $R_6$ eine Piperidingruppe ist.

11. Verfahren nach Anspruch 1 zur Herstellung von 6-(Hexahydro-4-methyl-1H-1,4-diazepin-1-yl)-3-methyl-7,8-tetramethylen-*s*-triazolo[4,3-*b*]pyridin.

**Revendications pour les Etats Contractants: BE CH DE FR IT LI LU NL SE**

1. Composé correspondant à la formule

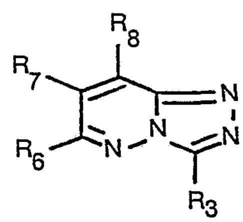

dans laquelle $R_3$ représente l'hydrogène ou un radical alkyle en $C_{1-4}$; $R_6$ représente un radical amino, (alkyl en $C_{1-4}$) amino, di-(alkyl en $C_{1-4}$)amino, amino hétérocyclique ou amino hétérocyclique substitué par un radical alkyle en $C_{1-4}$, où le fragment hétérocyclique forme un noyau pentagonal, hexagonal ou heptagonal, possédant un ou deux atomes d'azote dans le noyau, et zéro ou un atome de soufre ou d'oxygène dans le noyau; $R_7$ et $R_8$, pris ensemble représentent un enchaînement polyméthylène ou polyéthylène substitué constitué de 4 unités méthylène substituées par un radical alkyle en $C_{1-4}$, ou par des ponts méthano ou éthano; et les sels pharmacologiquement acceptables de ce composé.

2. Composé selon la revendication 1, dans lequel le composé correspond à la formule

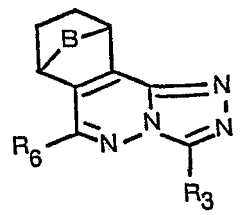

dans laquelle B représente le méthylène ou éthylène.

3. Composé selon la revendication 1, dans lequel $R_7$ et $R_8$ sont l'enchaînement tétraméthylène; $R_3$ est l'hydrogène, et $R_6$ est le radical 2-méthyl-1-pyrrolidinyle.

4. Composé selon la revendication 1, dans lequel $R_3$, $R_7$ et $R_8$ sont comme ils ont été définis dans la revendication 1, et $R_6$ représente un radical amino hétérocyclique ou amino hétérocyclique substitué par un radical alkyle en $C_{1-4}$, dans lequel le fragment hétérocyclique forme un noyau pentagonal, hexagonal ou heptagonal; et les sels pharmacologiquement acceptables de ce composé.

5. Composé selon la revendication 4, dans lequel $R_7$ et $R_8$ sont l'enchaînement tétraméthylène; $R_3$ est l'hydrogène, et $R_6$ est le radical hexahydro-1H-azépinyle-1.

6. Composé selon la revendication 4, dans lequel $R_7$ et $R_8$ sont l'enchaînement tétraméthylène; $R_3$ est l'hydrogène, et $R_6$ est le radical pyrrolidino.

7. Composé selon la revendication 4, dans lequel $R_7$ et $R_8$ sont l'enchaînement tétraméthylène; $R_3$ est l'hydrogène, et $R_6$ est le radical pipéridino.

8. Composé selon la revendication 1, qui est la 6-(hexahydro-4-méthyl-1H-1,4-diazépinyl-1)-3-méthyl-7,8-tétraméthylène-*s*-triazolo[4,3-*b*]pyridine.

9. Composition bronchodilatatrice comprenant une quantité efficace d'un composé selon la revendication 1, en mélange avec un excipient pharmaceutiquement acceptable.

10. Procédé pour la préparation d'un composé selon la revendication 1, qui consiste à faire réagir une 3,6-dihalogéno-pyridazine substituée en 4,5, ayant la formule

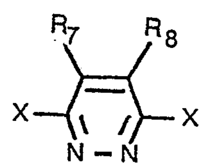

dans laquelle $R_7$ et $R_8$ sont comme ils ont été définis ci-dessus, et X représente les radicaux fluoro, chloro ou bromo, sur une amine corresponding au radical $R_6$ identifié ci-dessus, dans un solvant inerte ayant un point d'ébullition de 40°C à 200°C, en présence d'une base inorganique ou d'une amine non-nucléophile, ou d'un excès de l'amine $R_6$ en tant qu'acceptateur d'halogénure d'hydrogène, à une température de 25°C à 150°C, pour former une 3-halogénopyridazine, laquelle est mise ensuite à réagir avec un composé ayant la formule

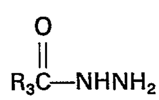

dans laquelle $R_3$ est comme il a été identifié ci-dessus, dans un milieu liquid inerte à une température de 50°C à 200°C; ou un composé de formule

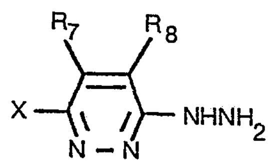

dans laquelle $R_7$ et $R_8$ sont comme ils ont été identifiés ci-dessus, et X est le radical fluoro, chloro ou bromo, est mis à réagir à une température de 40°C à 150°C sur un composé de formule

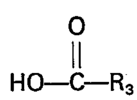

dans laquelle $R_3$ est comme il a été identifié ci-dessus, dans un excès d'un acide facultativement dilué avec un solvant inerte ayant un point d'ébullition de 40°C à 200°C, et la 6-halogénotriazolopyridazine obtenue est mise ensuite à réagir sur une amine correspondant au radical $R_6$ mentionné ci-dessus, à une température de 75°C à 160°C, en présence d'une base inorganique ou d'une amine non-nucleophile ou d'un excès de l'amine $R_6$ servant d'accepteur d'halogénure d'hydrogène dans un solvant inerte dont le point d'ébullition

est de 75°C à 200°C, ou sur un excès de la même amine $R_6$ en tant que milieu réactionnel à la température d'ébullition.

11. Procédé selon la revendication 10, dans lequel le solvant inerte est un solvant organique inerte.

12. Procédé selon la revendication 10, dans lequel le solvant inerte est un solvant hydroxylé.

13. Procédé selon la revendication 10, dans lequel on utilise un excés de la même amine $R_6$ en tant que milieu réactionnel à la température d'ébullition.

**Revendications pour l'Etat Contractant: AT**

1. Procédé pour la préparation d'un composé ayant la formule

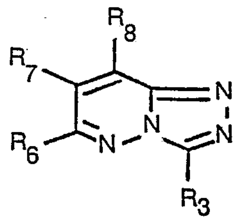

dans laquelle $R_3$ représente l'hydrogène ou un radical alkyle en $C_{1-4}$; $R_6$ représente un radical amino, (alkyl en $C_{1-4}$) amino, di-(alkyl en $C_{1-4}$)amino, amino hétérocyclique ou amino hétérocyclique substitué par un radical alkyle en $C_{1-4}$, où le fragment hétérocyclique forme un noyau pentagonal, hexagonal ou heptagonal, possédant un ou deux atomes d'azote dans le noyau, et zéro ou un atome de soufre ou d'oxygène dans le noyau; $R_7$ et $R_8$, pris ensemble, représentent un enchaînement polyéthylène ou polyméthylène substitué constitué de quatre unités méthylène substituées par un radical alkyle en $C_{1-4}$, ou par des ponts méthano ou éthano; et les sels pharmacologiquement acceptables de ce composé; qui consiste à faire réagir une 3,6-dihalogéno-pyridazine substituée en 4,5, ayant la formule

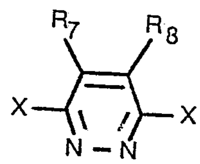

dans laquelle $R_7$ et $R_8$ sont comme ils ont été définis ci-dessus, et X représente les radicaux fluoro, chloro ou bromo, sur une amine correspondant au radical $R_6$ identifié ci-dessus, dans un solvant inerte ayant un point d'ébullition de 40°C à 200°C, et en présence d'une base inorganique ou d'une amine non-nucléophile, ou d'une excès de l'amine $R_6$ en tant qu'accepteur d'halogénure d'hydrogène, à une température de 25°C à 150°C, pour former une 3-halogénopyridazine laquelle est mise ensuite à réagir avec un composé ayant la formule

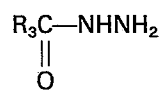

dans laquelle $R_3$ est comme il a été identifié ci-dessus, dans un milieu liquide inerte à une température de 50°C à 200°C; ou bien un composé de formule

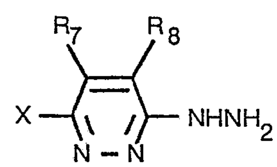

dans laquelle $R_7$ et $R_8$ sont comme ils ont été identifiés ci-dessus, et X est le radical fluoro, chloro ou bromo, est mis à réagir à une température de 40°C à 150°C sur un composé de formule

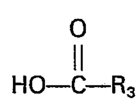

dans laquelle $R_3$ est comme il a été identifié ci-dessus, dans un excès d'un acide facultativement dilué avec un solvant inerte ayant un point d'ébullition de 40°C à 200°C, et la 6-halogénotriazolopyridazine obtenue est mise ensuite à réagir sur une amine correspondant au radical $R_6$ mentionné ci-dessus, à une température de 75°C à 160°C, en présence d'une base inorganique ou d'une amine non-nucléophile ou d'un excès de l'amine $R_6$ servant d'accepteur d'halogénure d'hydrogène dans un solvant inerte dont le point d'ébullition

est de 75°C à 200°C, ou sur un excès de la même amine $R_6$ en tant que milieu réactionnel à la température d'ébullition.

2. Procédé selon la revendication 1, dans lequel le solvant inerte est un solvant organique inerte.

3. Procédé selon la revendication 1, dans lequel le solvant inerte est un solvant hydroxylé.

4. Procédé selon la revendication 1, dans lequel on utilise un excès de la même amine $R_6$ en tant que milieu réactionnel à la température d'ébullition.

5. Procédé selon la revendication 1 pour préparer un composé de formule I, dans laquelle $R_6$ représente un radical amino hétérocyclique ou amino hétérocyclique substitué par un groupe alkyle en $C_{1-4}$, dans lequel le fragment hétérocyclique forme un noyau pentagonal, hexagonal ou heptagonal.

6. Procédé selon la revendication 5, dans lequel $R_6$ est le radical 2-méthyl-1-pyrrolidinyle.

7. Procédé selon la revendication 6, dans lequel $R_7$ et $R_8$ sont l'enchaînement tétraméthylène et $R_3$ est l'hydrogène.

8. Procédé selon la revendication 5, dans lequel $R_7$ et $R_8$ sont l'enchaînement tétraméthylène, $R_3$ est l'hydrogène et $R_6$ est le radical pyrrolidino.

9. Procédé selon la revendication 5, dans lequel $R_7$ et $R_8$ sont l'enchaînement tétraméthylène, $R_3$ est l'hydrogène et $R_6$ est le radical hexahydro-1H-azépinyle-1.

10. Procédé selon la revendication 5, dans lequel $R_7$ et $R_8$ sont l'enchaînement tétraméthylène, $R_3$ est l'hydrogène et $R_6$ est le radical pipéridino.

11. Procédé selon la revendication 1 pour la préparation de 6-(hexahydro-4-méthyl-1H-1,4-diazépinyl-1)-3-méthyl-7,8-tétraméthylène-*s*-triazolo[4,3-*b*]pyridine.